# EUROPEAN PATENT APPLICATION

(11) **EP 1 721 883 A1**
(43) Date of publication of application: **15.11.2006**
(21) Application number: 04792052.5
(22) Date of filing: 05.10.2004
(51) Int. Cl.: C07C 51/43, C07C 57/07

(54) **(METH)ACRYLIC ACID COMPOSITION AND PROCESS FOR PRODUCING THE SAME**

(30) Priority: 03.03.2004 JP 2004058779
(71) Applicant: MITSUBISHI CHEMICAL CORPORATION, Tokyo 108-0014 (JP)
(72) Inventor: YADA, Shuhei; C/O MITSUBISHI CHEMICAL CORPORATION, Minato-ku Tokyo 108-0014 (JP); TAKASAKI, Kenji; C/O MITSUBISHI CHEMICAL CO., Mie 5108530 (JP); OGAWA, Yasushi; C/O MITSUBISHI CHEMICAL CO., Mie 5108530 (JP); SUZUKI, Yoshiro; C/O MITSUBISHI CHEMICAL CO., Mie 5108530 (JP)
(74) Representative: HOFFMANN EITLE
(86) International application number: PCT/JP2004/014641
(87) International publication number: WO 2005/085165

(57) **Abstract**

The present invention removes impurities inhibiting storage stability of (meth)acrylic acid or stability in polymerization of (meth)acrylic acid when (meth)acrylic acid is used as a raw material, to thereby stabilize (meth)acrylic acid. A total concentration of aromatic hydrocarbons each having 8 to 10 carbon atoms in a (meth)acrylic acid composition is controlled to less than 2 ppm. The (meth)acrylic acid composition can be obtained by crystallizing (meth)acrylic acid from a crude (meth)acrylic acid composition containing the aromatic hydrocarbons, sweating the crystallized (meth)acrylic acid, and separating the (meth)acrylic acid remained as a crystal after the sweating.

## Description

### Technical Field

The present invention relates to a method for producing a high purity (meth) acrylic acid composition, and more specifically to a method for producing a high purity (meth) acrylic acid composition through crystallization of a crude (meth)acrylic acid composition containing as impurities aromatic hydrocarbons each having 8 to 10 carbon atoms.

In the present description, the notation "(meth) acrylic acid" refers to one or both of acrylic acid and methacrylic acid.

### Background Art

Acrylic acid is produced through a following method, for example. That is, acrylic acid is produced by: subjecting propylene as a raw material to vapor-phase catalytic oxidation; absorbing an obtained oxidation product in water to collect acrylic acid as an aqueous solution; concentrating the obtained aqueous solution in the presence of an azeotropic solvent to obtain crude acrylic acid; and purifying the crude acrylic acid in a distillation column to collect high purity acrylic acid. Methacrylic acid is produced through a similar process as that described above by using isobutylene as a raw material.

In the production process, aldehydes such as furfural and benzaldehyde, and carboxylic acids such as maleic acid, maleic anhydride, and acetic acid are known to be produced as by-products.

A demand of acrylic acid has increased recently as a raw material for super absorbent polymers used in disposable diapers and the like and for food additives, and high purity acrylic acid is demanded in such applications. That is, use of crude acrylic acid as a raw material for polymerization without removing impurities causes problems in stable production of products employing acrylic acid as a raw material. The problems include drastic fluctuation in polymerization induction period, reduction in degree of polymerization of a polymer to be obtained, and coloring of a polymerized product.

However, removal of impurities present in crude acrylic acid that is obtained through vapor-phase catalytic oxidation through distillation is not easy, requiring an increase in theoretical plate number (increase of 6 to 20 plates, for example), an increase in reflux ratio, and the like.

Examples of a conventional method of producing high purity acrylic acid by separating and removing aldehydes from crude acrylic acid obtained through vapor-phase catalytic oxidation include: a method making crude (meth)acrylic acid react in the presence of a compound (aldehyde removing agent) having one or more mercapto groups in a molecule and an acid catalyst (see JP 04-029658 B, for example); and a method involving distilling crude (meth)acrylic acid in the presence of hydrazines (see JP 49-030312 A and JP 58-037290 B, for example).

Such methods enabled separation of impurities each modified to have a high boiling point through chemical treatment, but were not sufficient for separation of aromatic hydrocarbons each having 8 to 10 carbon atoms.

In particular, waste water obtained after the separation of acrylic acid in a distillation step has been recycled recently as a collecting liquid brought into contact with a gaseous oxidation product obtained through vapor-phase catalytic oxidation of propylene or the like, for reduction of production cost. However, by-products of an oxidation reaction or by-products of compounds used as auxiliaries are accumulated in the waste water. Thus, the by-products are circulated through the production process as an operating period extends and the number of recycling increases.

Such compounds are discharged from the process when an operation of the distillation column becomes unstable in the production process or the like, and may be distilled along with acrylic acid, which is a product. Such impurities may become a cause of deteriorating storage stability of acrylic acid, and a cause of inhibiting polymerization of acrylic acid when acrylic acid is used as a raw material for a polymerization reaction.

### Disclosure of the Invention

The present invention has been made in view of the above circumstances, and is aimed at stabilizing (meth)acrylic acid by removing impurities.

The inventors of the present invention have analyzed in detail and found out that the stability of acrylic acid deteriorates in the presence of aromatic hydrocarbons each having 8 to 10 carbon atoms. The inventors of the present invention have conducted extensive studies, have found out that separation of aromatic hydrocarbons each having 8 to 10 carbon atoms can be easily attained through crystallization, and thus have completed the present invention.

That is, the present invention relates to: a(meth)acrylic acid composition wherein a total concentration of aromatic hydrocarbons each having 8 to 10 carbon atoms is less than 2 ppm; and a method of producing the (meth)acrylic acid composition by using crystallization of (meth)acrylic acid.

### Best Mode for carrying out the Invention

The (meth)acrylic acid composition of the present invention contains aromatic hydrocarbons each having 8 to 10 carbon atoms at a total concentration of less than 2 ppm.

In the present invention, the (meth)acrylic acid composition refers to a composition obtained (meth) acrylic acid in a crystal state through crystallization. The (meth)acrylic acid composition includes (meth)acrylic acid itself and (meth) acrylic acid having trace impurities.

The (meth)acrylic acid composition of the present invention can be produced by crystallizing (meth)acrylic acid from a crude (meth) acrylic acid composition containing aromatic hydrocarbons each having 8 to 10 carbon atoms, sweating the crystallized (meth)acrylic acid, and separating the (meth)acrylic acid remained as a crystal after the sweating.

Hereinafter, the present invention will be described in detail.

In a production process for (meth)acrylic acid, an oxidation product is first produced through vapor-phase catalytic oxidation of propane, propylene, acrolein, or isobutene. The obtained oxidation product contains target acrylic acid or methacrylic acid. In addition, the obtained oxidation product contains as impurities: aldehydes such as furfural, formaldehyde, acetaldehyde, and benzaldehyde; ketones such as acetone; dibasic acids such as maleic acid; and a large volume of water.

The oxidation product is supplied to a dehydration step to separate and remove water from a (meth) acrylic acid component. The dehydration step employs dehydration through a method such as a solvent extraction method or an azeotropic distillation method. Examples of an azeotropic solvent used at this time include: esters which boil together with water such as methyl acetate, ethyl acetate, propyl acetate, isopropyl acetate, and isobutyl acetate; ketones which boil together with water such as methyl ethyl ketone and methyl isobutyl ketone; aromatic hydrocarbons which boil together with water or acetic acid such as toluene, ethylbenzene, and xylene; and mixtures thereof.

In the solvent extraction method, (meth) acrylic acid is extracted from an aqueous solution of (meth) acrylic acid into an extraction solvent. Examples of the extraction solvent include methyl isobutyl ketone, isopropyl acetate, methyl ethyl ketone, toluene, and cyclohexane. A (meth) acrylic acid extract contains a small amount of water, and thus water is generally removed from the (meth) acrylic acid extract through the azeotropic distillation method. In such a case, the same type of solvent as the azeotropic solvent is preferably used as the extraction solvent from the viewpoint of simplification of the process.

For example, a vacuum distillation column is usually used as an azeotropic distillation column. When taking this as an example, Low boiling point components in the impurities, that is, formaldhyde, acetaldehyde, acetone, and the like are distilled along with water, but impurities in the azeotropic solvent added as the azeotropic solvent are concentrated in the distillation column. The concentrated compounds are distilled out of the column along with a non-condensable gas in the distillation column through a pressure control system of the distillation column.

The inventors of the present invention have conducted extensive studies and have found a compound which could not be detected in a raw material, but which is a component concentrated while circulating the production process for (meth) acrylic acid, and which is distilled in a high purity (meth)acrylic acid composition to destabilize polymerization when (meth)acrylic acid is used as a raw material.

The impurities in the present invention refer to aromatic hydrocarbons each having 8 to 10 carbon atoms. Examples of the aromatic hydrocarbons each having 8 to 10 carbon atoms include 3-membered, 4-membered, 5-membered, or 6-membered ring aromatic monocyclic hydrocarbons each having an alkyl group into which a hydrogen atom bonded to the ring was substituted. Preferable examples thereof include 6-membered aromatic ring monocyclic hydrocarbons each having an alkyl group into which a hydrogen atom bonded to the ring was substituted. In the present invention, the aromatic monocyclic hydrocarbons each refer to a compound comprised of one aromatic ring and a hydrocarbon group bonded thereto.

Examples of the alkyl group include a methyl group, an ethyl group, an n-propyl group, an i-propyl group, an n-butyl group, an i-butyl group, and a t-butyl group. The number of substitution by the alkyl group is preferably 1 to 4.

The aromatic hydrocarbons described above each have 8 to 10 carbon atoms, preferably 9 to 10 carbon atoms. Examples of the aromatic hydrocarbons each having 9 to 10 carbon atoms are represented by the following structural formulae and specifically include 2-ethyltoluene, 3-ethyltoluene, 4-ethyltoluene, n-propylbenzene, 1,2,3-trimethylbenzene, 1,2,4-trimethylbenzene, 1,3,5-trimethylbenzene, isopropylbenzene, 1,2,3,5-tetramethylbenzene, 1,2,3,4-tetramethylbenzene, 1,2,4,5-tetramethylbenzene, 4-ethyl-m-xylene, 5-ethyl-m-xylene, 2-ethyl-p-xylene, 2-n-propyltoluene, 3-n-propyltoluene, 4-n-propyltoluene, 1,2-diethylbenzene, 1,3-diethylbenzene, 1,4-diethylbenzene, sec-butylbenzene, tert-butylbenzene, o-cymene, m-cymene, and p-cymene.

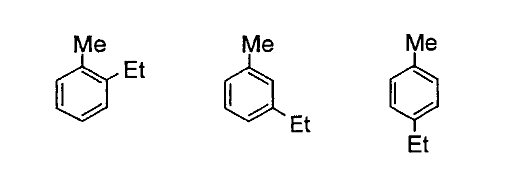

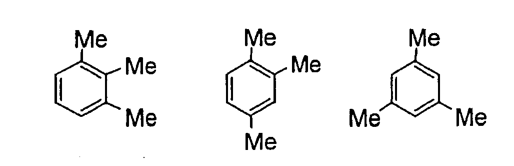

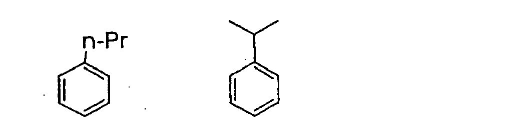

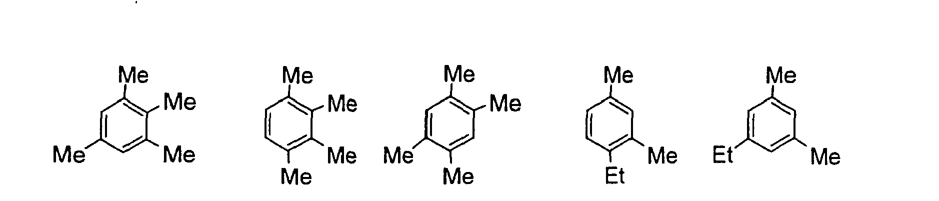

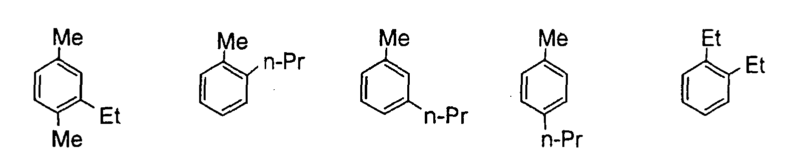

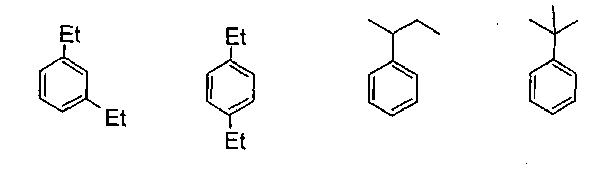

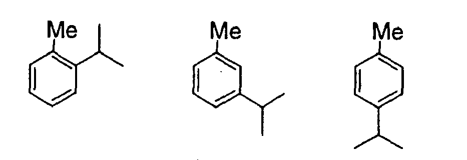

The impurities are hardly separated, because they are isomers having similar numbers of carbon atoms, and are presumably present in a mixed state.

A preferable method of removing aromatic hydrocarbons each having 8 to 10 carbon atoms includes purification through crystallization of (meth)acrylic acid. That is, the purification through crystallization of (meth)acrylic acid involves: crystallizing (meth)acrylic acid from a crude (meth)acrylic acid composition containing aromatic hydrocarbons each having 8 to 10 carbon atoms; sweating the crystallized (meth)acrylic acid; and separating the (meth)acrylic acid remained as a crystal after the sweating.

As the crude (meth)acrylic acid composition, a composition, obtained by distilling a (meth)acrylic acid water-containing composition containing (meth)acrylic acid and water in the presence of an azeotropic solvent to remove water from the (meth)acrylic acid water-containing composition, may be employed. As the crude (meth)acrylic acid composition, the (meth)acrylic acid water-containing composition may be employed. Examples of the (meth)acrylic acid water-containing composition include: an aqueous solution of (meth) acrylic acid which may contain an organic solvent; and an organic solution of (meth)acrylic acid which may contain water. A specific example of the crude (meth)acrylic acid composition includes a composition obtained through the azeotropic distillation method or solvent extraction method in which the above-mentioned azeotropic solvent or extraction solvent are repeatedly used.

In the present invention, crystallization of (meth)acrylic acid can be conducted by lowering a temperature of a (meth)acrylic acid composition before crystallization. An operating temperature for crystallization only needs to be equal to or lower than a temperature at which a crystal is separated, but is preferably - 10 to 13°C from the operational standpoint.

In the present invention, sweating of the crystallized (meth) acrylic acid can be conducted by melting part of the crystallized (meth)acrylic acid. The sweating refers to a phenomenon and operation for removing impurities, which result from a mother liquor incorporated in a crystal, out of the crystal by increasing a temperature of the crystal and the like.

Such an operation can be conducted by gradually melting the crystallized (meth) acrylic acid. Such gradual melting can be conducted by mildly increasing a temperature of the crystallized (meth)acrylic acid, by bringing the crystallized (meth)acrylic acid into contact with a mother liquor, or by bringing the crystallized (meth)acrylic acid into contact with a melted product of the (meth)acrylic acid crystal.

Further, the (meth)acrylic acid crystal separated after the sweating may be melted, and a process involving crystallization of (meth)acrylic acid, sweating of the crystallized (meth)acrylic acid, and separation of the (meth)acrylic acid may be repeated. Such an operation is preferable from the viewpoint of further removing various impurities such as the aromatic monocyclic hydrocarbons to obtain higher purity (meth)acrylic acid.

A device for conducting crystallization or sweating is not particularly limited so long as the device can avoid abrupt application of heat during melting of (meth) acrylic acid. In case of small-scale, simple equipment such as a glass vessel with a cooling thermostatic bath may be employed. In case of industrial-scale, a known device described in "Chemical Engineering Handbook, 6th edition (1999)" may be employed, for example. Crystallization or sweating may be a batch operation or a continuous operation.

A method of separating a crystal and a mother liquor is not particularly limited so long as a solid and a liquid can be separated with the method. A known method such as filtration or centrifugation can be used. Separation may be a batch operation or a continuous operation.

For the crystallization and sweating of the (meth)acrylic acid and the separation of the crystal and the mother liquor, a device capable of conducting all of the operations is preferably used. Examples of such a device include: a batch-type falling film device (FFC); a continuous-type 4C crystallizer having a plurality of crystallization tanks and a column-type purification portion; and a KCP device described in C. Shimizu, "Purification in organic synthesis by Kureha Continuous Crystal Purifier", Chemical Engineering, 27, 3, 49 (1982).

The separated crystal may be taken out in a crystal state from the device described above, or may be melted into a liquid state and then taken out from the device.

A total concentration of the aromatic hydrocarbons in the (meth)acrylic acid composition can be measured through a known quantitative determination method. Examples of such a measurement method include an absolute calibration method and an internal standard method employing high-performance liquid chromatography or gas chromatography. In the measurement, a measurement sample, subjected to treatment for appropriately measuring a concentration of each of the aromatic hydrocarbons in the measurement sample by concentrating the sample or the like according to detection intensity of each of the aromatic hydrocarbons, may be employed.

The separated mother liquor may be used as it is for a raw material in production of (meth)acrylate or may be returned to a production process for (meth)acrylic acid. The separated mother liquor is preferably used as it is for a raw material in production of (meth)acrylate without particular repurification for economical reasons.

According to the present invention described above, adjusting a total concentration of the aromatic hydrocarbons each having 8 to 10 carbon atoms in (meth)acrylic acid to less than 2 ppm can stabilize (meth) acrylic acid during storage and eliminate effects in polymerization of the (meth)acrylic acid used as a raw material for polymerization, and thus, the present invention has a significant industrial value.

### Examples

Hereinafter, the present invention will be described in more detail with reference to examples, but the present invention is not limited to the following examples without departing from the gist of the present invention.

Acrylic acid used in each of examples and comparative examples is obtained through vapor-phase catalytic oxidation of propylene and acrolein using molecular oxygen, condensation of a reaction product gas, extraction thereof, and distillation of the extract, unless otherwise noted.

### <Example 1>

Acrylic acid used as a raw material contained: 1 mass ppm of 2-ethyltoluene; 5 mass ppm of 1,2,4-trimethylbenzene; 25 mass ppm of 3-n-propyltoluene; 9 mass ppm of 5-ethyl-m-xylene; 13 mass ppm of 2-n-propyltoluene;2massppm of 2-ethyl-p-xylxne; 607 mass ppm of acetic acid; 385 mass ppm of propionic acid; 1,310 mass ppm of water; 1,600 mass ppm of acrylic acid dimer; and 200 mass ppm of methoxyhydroquinone.

1 L of acrylic acid was gradually cooled in a refrigerator at 5° C for 15 hours, and was gradually melted at room temperature until a volume of crystallized acrylic acid became 500 mL.

The remaining crystallized acrylic acid was filtered and separated, and the total amount of the separated crystallized acrylic acid was melted. The melted acrylic acid was similarly cooled for crystallization, and was gradually melted at room temperature until a volume of crystallized acrylic acid became 250 mL.

In this way, a procedure involving melting a half amount of the acrylic acid crystallized through gradual cooling as described above and taking out a half amount of the crystallized acrylic acid was repeated twice, to thereby obtain 250 mL of high purity acrylic acid containing 322 mass ppm of acetic acid, 263 mass ppm of propionic acid, 180 mass ppm of water, 115 mass ppm of acrylic acid dimer, and 5 mass ppm of methoxyhydroquinone, before purification.

20 mL of the high purity acrylic acid was weighed into a sample bottle using a whole pipette, and was left to stand isothermally for 3 months in a thermostat at 17°C. After 3 months, acrylic acid was retained in a liquid state.

### <Comparative Example 1>

Added to the high purity acrylic acid obtained in Example 1 were 1 mass ppm of 2-ethyltoluene, 5 mass ppm of 1,2,4-trimethylbenzene, 25 mass ppm of 3-n-propyltoluene, 9 mass ppm of 5-ethyl-m-xylene, 13 mass ppm of 2-n-propyltoluene, and 2 mass ppm of 2-ethyl-p-xylene, which were present before the purification. The mixture was weighed into a sample bottle and left to stand isothermally for 3 months in a thermostat at 17°C in the same manner as in Example 1.

Property of the mixture was determined after 3 months. As a result, the acrylic acid left to stand was no longer in a liquid state, but was a clear and colorless polyacrylic acid polymer.

### Industrial Applicability

The present invention provides (meth) acrylic acid in specific spec.. The (meth)acrylic acid can be stored stably in a liquid state. Further, when polymerization is conducted using the (meth)acrylic acid as a raw material, inhibition of polymerization or destabilization of polymerization can be prevented. The value of the (meth)acrylic acid in specific spec. of the present invention appears particularly and significantly in a process for producing a (meth)acrylic acid polymer such as a super absorbent polymer requiring high quality (meth)acrylic acid, for example.

## Claims

1. A (meth)acrylic acid composition, wherein a total concentration of aromatic hydrocarbons each having 8 to 10 carbon atoms is less than 2 ppm.

2. The (meth)acrylic acid composition according to claim 1, wherein the aromatic hydrocarbons each have 9 to 10 carbon atoms and the aromatic hydrocarbons each comprise one or more compounds selected from 6-membered ring aromatic monocyclic hydrocarbons each having an alkyl group into which a hydrogen atom bonded to the ring was substituted.

3. A method for producing a (meth)acrylic acid composition from a crude (meth)acrylic acid composition comprising aromatic hydrocarbons each having 8 to 10 carbon atoms, in which a total concentration of the aromatic hydrocarbons in the composition is less than 2 ppm, wherein the method comprises:
crystallizing (meth)acrylic acid from the crude (meth) acrylic acid composition; sweating the crystallized (meth)acrylic acid; and separating the (meth)acrylic acid remained as a crystal after the sweating.

4. The method for producing a (meth)acrylic acid composition according to claim 3, wherein the method comprising: crystallizing (meth)acrylic acid from the separated (meth)acrylic acid, and carrying out a step of further performing the sweating of the crystallized (meth)acrylic acid and the subsequent separation.

5. The method for producing a (meth)acrylic acid composition according to claim 3, wherein a composition obtained by distilling a (meth)acrylic acid water-containing composition which comprises (meth)acrylic acid and water in the presence of an azeotropic solvent which boils together with water, to remove water from the (meth)acrylic acid water-containing composition is used as the crude (meth)acrylic acid composition.

6. The method for producing a (meth)acrylic acid composition according to claim 3, wherein the aromatic hydrocarbons each have 9 to 10 carbon atoms and the aromatic hydrocarbons each comprise one or more compounds selected from 6-membered ring aromatic monocyclic hydrocarbons each having an alkyl group into which a hydrogen atom bonded to the ring was substituted.
